# NEUE EUROPÄISCHE PATENTSCHRIFT

(11) **EP 0 906 340 B2**
(45) Veröffentlichungstag und Bekanntmachung des Hinweises auf die Entscheidung über den Einspruch: **03.09.2008**
(45) Hinweis auf die Patenterteilung: 23.06.2004
(21) Anmeldenummer: 97922744.4
(22) Anmeldetag: 09.04.1997
(51) Int. Cl.: C07K 14/755, C07K 1/22

(54) **REINIGUNG VON FAKTOR VIII-KOMPLEX DURCH IMMUNAFFINITÄTSCHROMATOGRAPHIE**
PURIFICATION OF FACTOR VIII COMPLEX BY IMMUNOAFFINITY CHROMATOGRAPHY
PURIFICATION DU COMPLEXE DU FACTEUR VIII PAR CHROMATOGRAPHIE D'IMMUNOAFFINITE

(30) Priorität: 12.04.1996 AT 66796
(43) Veröffentlichungstag der Anmeldung: 07.04.1999
(73) Patentinhaber: Baxter Aktiengesellschaft, 1221 Wien (AT)
(72) Erfinder: SCHÖNHOFER, Wolfgang, A-3100 St. Pölten (AT); EIBL, Johann, A-1180 Wien (AT); WEBER, Alfred, A-1210 Wien (AT); LINNAU, Yendra, A-1220 Wien (AT)
(74) Vertreter: Alge, Daniel
(86) Internationale Anmeldenummer: PCT/AT1997/000069
(87) Internationale Veröffentlichungsnummer: WO 1997/039033

(56) Entgegenhaltungen:
- EP-A- 0 281 089
- WO-A-86/01718
- PATENT ABSTRACTS OF JAPAN vol. 013, no. 402 (C-633), 6.September 1989 & JP 01 144991 A (KAGAKU OYOBI KETSUSEI RIYOUHOU KENKYUSHO), 7.Juni 1989,
- THROMB. RES. (UNITED STATES) , Bd. 74, Nr. 4, 15.Mai 1994, Seiten 347-354, XP002039902 KOOPS ET AL.: "Factor VIII:C assay: influence of buffer components used in immunoaffinity chromatography for purification of factor VIII/von Willebrand factor"
- THROMB HAEMOST , Bd. 57, Nr. 1, 3.Februar 1987, GERMANY, WEST, Seiten 102-105, XP002039903 HORNSEY VS ET AL.: "Immunoaffinity purification of factor VIII complex." in der Anmeldung erwähnt
- ANN. CLIN. LAB. SCI. , Bd. 19, Nr. 2, 1989, UNITED STATES, Seiten 84-91, XP002039904 WEINSTEIN R.E.: "Immunoaffinity purification of factor VIII"

## Beschreibung

Die vorliegende Erfindung betrifft ein Verfahren zur Herstellung eines hochgereinigten Komplex bestehend aus den Komponenten Faktor VIII (Faktor VIII:C oder FVIII) und von Willebrand-Faktor(vWF), und einer stabilen pharmazeutischen Präparation, enthaltend den hochgereinigten Komplex.

Von Willebrand-Faktor ist ein multimeres Glykoprotein, welches von einem Gen auf Chromosom 12 codiert wird und im Plasma in Konzentrationen von 5 bis 10 µg/ml frei und als nicht-kovalenter Komplex mit Gerinnungsfaktor VIII, dem Protein, welches durch ein Gen auf Chromosom 10 codiert wird und in Haemophilie A defekt ist bzw. fehlt, zirkuliert.

Zu den zwei wichtigsten Funktionen von vWF in der Haemostase zählen:
1) die Adhäsion der Thrombozyten an verletztes Endothel, wobei vWF an das verwundete Sub-Endothelium bindet, und eine Brücke zwischen dieser Oberfläche und den Blutplättchen sowie eine Aggregation der Plättchen untereinander ermöglicht. Die erste Interaktion zwischen Thrombozyten und Subendothel erfolgt über das Glykoprotein Ib der Thrombozytenmembran und den Kollagenfasern des verletzten Endothels. An diesen beiden Proteinen bindet vWF und vermittelt so die Bildung einer ersten Thrombozytenschicht. Die weitere Vernetzung der Blutplättchen untereinander vermittelt vWF durch die Bindung an den Glykoproteinkomplex IIb/IIIa. Für diese Aufgaben der primären Haemostase sind hauptsächlich die großen Multimeren verantwortlich. (Eller; Lab. Med. (1994); 18: 168-176)
2) durch seine Bindungsstelle für FVIII beeinflußt vWF auch die plasmatische Gerinnung. FVIII liegt im Plasma fast ausschließlich in einem nicht-kovalenten Komplex mit vWF vor, wobei etwa jedes zehnte vWF-Molekül ein FVIII-Molekül trägt. Als Träger dienen vor allem Dimere und kleine Multimere. Durch diese Komplexierung mit vWF ist FVIII vor einer verstärkten proteolytischen Inaktivierung (z.B. durch aktiviertes Protein C) geschützt.

Desweiteren wird FVIII durch die Komplexbildung hinsichtlich seiner Cofaktoraktivität in der intrinsischen Gerinnung potenziert (Eller; Lab. Med. (1994); 18: 168-176).

vWF wird in den vaskulären Endothelzellen, welche die Hauptquelle dieses Plasmaproteins bilden, durch konstitutive oder stimulierte Freisetzung gebildet, aber auch in einem geringeren Anteil durch die Megakariozyten synthetisiert (PNAS 92 (1995), 2428-2432).

Das primäre Produkt der Translation besteht aus 2813 Aminosäuren. Nach Abspaltung des Signalpeptids (22 Aminosäuren) kommt es zur Dimerisierung. Die weitere Prozessierung erfolgt im Golgi-Apparat, wobei die Dimere unter Abspaltung des Propeptids (741 Aminosäuren) polymerisiert. Das Propeptid spielt bei der weiteren Verknüpfung der Dimere eine wichtige Rolle, wobei es die Ausbildung von Disulfidbrücken am aminoterminalen Ende katalysiert. Somit entwickeln sich unterschiedlich große Oligomere von der Größe eines Dimers mit 500.000 Dalton bis zu großen Multimeren mit bis zu 20 Millionen Dalton. Zusätzlich zu den proteolytischen Vorgängen ist der vMF noch anderen posttranslationalen Modifikationen unterworfen, einschließlich der Glykosylierung und Sulfatisierung. (Mancuso et al.; Hämostaseologie (1989); 9: 122-129)

Aus der Komplexizität der Biosynthese resultiert daher eine Vielzahl verschiedenster vWF-Moleküle mit unterschiedlichsten Aufgaben und Eigenschaften.

Dies führt dazu, daß von Willebrand-Faktor sehr unterschiedliche Bindungsaktivitäten zu seinen natürlichen Bindungspartnern aufweisen kann. Insbesondere zeigte sich, daß die Bindungen verschiedener vMF-Moleküle zum Glykoprotein Ib, zu Kollagen, zu Heparin, zum Glykoprotein IIb/IIIa-Komplex, zu Faktor VIII und zum Sub-Endothelium unterschiedlich stark sein können.

Dies bedeutet aber, daß jede vWF-Präparation aus einem Gemisch dieser verschiedenen vWF-Proteine bzw. vWF-Aggregate zusammengesetzt ist und daher bezüglich der Eigenschaften, wie die essentielle Bindungsstärke zu Faktor VIII, heterogen ist.

Das Auftreten von verschiedenen Formen von vWF begründet auch die komplexen und unterschiedlichen Phänotypen bei der Pathophysiologie der von Willebrand-Krankheit, welche in bestimmten Fällen auf eine Unter-, in anderen Fällen auf eine Überproduktion des von Willebrand-Faktors zurückzuführen ist. So führt z.B. eine Überproduktion von vWF zur vermehrten Neigung von Thrombosen, wohingegen eine Unterversorgung von vWF eine vermehrte Blutungsneigung oder verlängerte Blutungszeit zur Folge hat, jedoch ist dies keineswegs generell gültig, entscheidend ist nämlich auch, in welcher Form der von Willebrand-Faktor über- oder unterproduziert wird.

Zur Unterscheidung und Charakterisierung der Eigenschaften des vWF und des vWF-Syndroms werden eine Reihe von Analysenmethoden eingesetzt.

So ist für die Diagnostik die Ristocetin-Cofaktor-Aktivitätsbestimmung unentbehrlich. Dabei wird die Thrombozytenaggregation in Gegenwart des Antibiotikums Ristocetin untersucht, welche bei Patienten mit vWF-Syndrom vermindert oder überhaupt nicht vorhanden ist. (Macfarlane et al.; Thrombosis et Diathesis Haemorehagica 1775; 34: 306-308).

Desweiteren kann die Kollagenbindungsaktivität des vWF zur Differenzierung des vWF-Syndroms herangezogen werden (Thomas et al.; Hämastaseologie (1994); 14: 133-139).

Die Bindungsdissoziationskonstante zwischen vWF und FVIII kann nach der Methode von Vlot et al. bestimmt werden (Blood 83 (11) (1995); 3150-3157).

Der molekulare Aufbau des vWF wird durch Analyse der Multimerstruktur mittels einer SDS-Elektrophorese in 1,2% Agarosegelen bestimmt (Ruggeri et al.; Blood (1981); 57: 1140- 1143).

Zur Bestimmung der vWF-Antigen-Gesamtmenge werden verschiedenste kommerziell erhältliche ELISA-Testktis herangezogen.

Die Herstellung eines optimalen FVIII/vWF-Komplexes sollte sich zum Ziele setzen, ein stabiles, vor allem aber ein von unerwünschten Begleitproteinen freies Produkt zu liefern, da jede unnötige Proteinfracht das Risiko unerwünschter Nebenwirkungen in sich birgt.

Somit stellt jede Konzentration an vWF, die nicht zur Stabilisierung von FVIII notwendig ist, eine Belastung des Hämophilen dar.

Bisher konnte im Zuge von Herstellungsverfahren für von Willebrand-Faktor-Präparationen, insbesondere aus Plasmapools, das Risiko einer heterogenen Zusammensetzung auf Grund der verschieden auftretenden Formen des von Willebrand-Faktors nie ausgeschalten werden und es ist bisher im Stand der Technik nicht gelungen, eine vWF-Präparation mit einheitlichen Eigenschaften, beispielsweise bezüglich der Bindungsaktivität zu einem bestimmten Liganden, zu gewinnen.

Es war bekannt, Faktor VIII-Komplex mittels verschiedener antivWF-monoklonaler Antikörper sowohl aus Plasma als auch aus Kryopräzipitat zu reinigen (Thromb. Haemostas. 57 (1987), 102-105). Dabei wurde auch die Stabilität des FVIII/vWF-Komplexes in unterschiedlichen Puffern bei pH 6,5 getestet, darunter solche, die Glykole, Aminosäuren, Chaotrope, Amine, andere Salze oder organische Lösungsmittel und/oder Detergentien enthalten. Der Zusatz von Lysin zu Pufferlöungen mit chaotropen Stoffen in hoher Konzentration, wie z.B. 3M Harnstoff, bewirkte einen Schutz von Faktor VIII:C/vWF:Ag gegenüber denaturierenden Effekten. Die Aktivitäten von Faktor VIII:C und vWF R.cof. betrugen beispielsweise nach Inkubation mit 20 % V/V Ethylenglykol + 1 M KJ 72 % bzw. 48%, bei Behandlung mit 3 M Harnstoff 88% bzw. 77 %.

Die spezifische Aktivität von Faktor VIII:C in einem Endprodukt, eluiert mit 1 M KJ + 1 M Lysin + 20 mM Imidazol + 5 mM CaCl2, pH 6,5 betrug 45 I.E./mg Gesamtprotein, jene von vWF 60 I.E./mg.

Es wurde auch berichtet, Faktor VIII/vWF-Komplex aus Kryopräzipitat nach Adsorption an Al(OH)₃ und Durchführung einer Virusinaktivierung unter Verwendung von anti-vWF monoklonaler Antikörper chromatographisch zu reinigen (Biotechnol. Blood Prot. 227 (1993), 109-114). Die Elution des adsorbierten Komplexes erfolgte bei pH 6,5 unter Zusatz des chaotropen Agens KJ (1 M). Das Verhältnis Faktor VIII:C/vWF:AG betrug 0,8, die spezifische Aktivität von Faktor VIII:C 38 i.u./mg.

Schließlich ist aus der EP-0 295 645-A2 bekannt, Faktor VIII-Komplex mittels Affinitätschromatographie unter Verwendung spezifischer, gegen vWF gerichteter Peptide aus heterogenen biologischen Flüssigkeiten zu reinigen. Der Komplex wurde dabei unter Verwendung von pH-Gradienten oder Puffern mit hoher Ionenstärke eluiert (siehe Beispiel 5 der EP-0 295 645-A2).

In der EP 0 416 983 A1 wird eine Präparation mit dem Faktor VIII/vWF-Komplex beschrieben, welche durch Anionenaustauschchromatographie hergestellt wurde. Gemäß der WO 86/01718 A1 wurde eine Faktor VIII/vwF-Komplex-Präparation mittels Chromatographie an einem monoklonalen Antikörper erhalten.

In der JP 01/144991 A wird die Reinigung von Faktor VIII:C oder eines Faktor VIII:C/vWF-Komplexes mittels Adsorption an einen Träger mit einem monoklonalen Antikörper beschrieben, wobei Faktor VIII:C oder der Faktor VIII:C/vWF-Komplex bei einer niedrigen Metallionenkonzentration gebunden und bei einer hohen Metallionenkonzentration eluiert wird.

Allen diesen Faktor VIII/vWF-Komplexen im Stand der Technik ist gemein, daß sie trotz hoher Anreicherung und Aufreinigung der Präparate kein in bezug auf vWF homogenes Präparat, was seine Bindung gegenüber Faktor VIII:C anbelangt, zur Verfügung stellen konnten und daher kein nativer Faktor VIII/vWF-Komplex mit hoher spezifischer Aktivität existierte.

Die vorliegende Erfindung stellt sich daher zur Aufgabe, eine Faktor VIII/vWF-Präparation, enthaltend einen Faktor VIII/vWF-Komplex, zur Verfügung zu stellen, welcher in bezug auf die Bindungseigenschaften des vWF bezüglich des Faktor VIII eine einheitliche Struktur aufweist und daher besonders verträglich bzw. stabil ist.

Diese Aufgabe wird erfindungsgemäß durch das in den Ansprüchen 1 - 10 beschriebene Verfahren zur Herstellung einer Präparation, enthaltend einen hochgereinigten Komplex, bestehend aus den Komponenten Faktor VIII und von Willebrand-Faktor mit einer spezifischen Aktivität von mindestens 70, vorzugsweise 100 bis 300 E Faktor VIII:C/mg gelöst, welcher durch Reinigen eines Ausgangsmaterials, enthaltend Faktor VIII:C und vWF, mittels Affinitätschromatographie erhältlich ist, wobei der Komplex aus Faktor VIII:C und vWF sowie der nicht komplexierte Faktor VIII:C bzw. vWF fraktioniert eluiert werden. Dabei wird nicht-komplexierter, also überschüssiger, Faktor VIII:C bzw. von Willebrand-Faktor durch Immunaffinitätschromatographie abgetrennt, insbesondere durch die fraktionierte Elution, bei der der komplexierte und nicht-komplexierte Faktor in getrennten Fraktionen erhalten wird.

Durch den erfindungsgemäßen Abtrennungsschritt von nicht-komplexiertem Faktor VIII:C bzw. von Willebrand-Faktor werden gezielt insbesondere von Willebrand-Faktor-Moleküle mit beeinträchtigter Affinität zu Faktor VIII abgetrennt, und es wird erstmalig ein homogener, nativer Faktor VIII/vWF-Komplex erhalten, der eine bezüglich der Affinität zu Faktor VIII definierte Fraktion des vWF enthält.

Der erfindungsgemäß erhaltene Komplex ist überraschenderweise bei der Immunaffinitätschromatographie in einem chaotropen Milieu beständig. Sogar in einem Medium mit einer Leitfähigkeit von bis zu 30 mS, vorzugsweise bis zu 40 mS, ist keine Dissoziation des Komplexes festzustellen. So kann der stabile Komplex des Faktor VIII und vMF von der anti-vMF-Säule sogar bei einer relativ hohen Ionenstärke entsprechend einer 2- bis 3-fach isotonen Lösung, eluiert und gewonnen werden.

Im erfindungsgemäß erhaltenen Komplex weist der vWF vorzugsweise eine Kollagenbindungsaktivität im Bereich von 0,2 bis 0,6, bezogen auf das vWF-Antigen (Plasmaeinheit/Plasmaeinheit), auf.

Der erfindungsgemäß erhaltene Komplex ist vorzugsweise erhältlich durch Reinigen eines die beiden Komponenten enthaltenden Ausgangsmaterials, wobei der Komplex sowie der separate Faktor VIII:C bzw. vWF vom Immunaffinitätsträger fraktioniert eluiert werden.

Die Elutionsmittel können auch relativ hohe Konzentrationen an Chaotropen bzw. chaotrop wirksamen Salzen enthalten, da die Immunaffinitätschromatographie vorzugsweise an Antikörpern vorgenommen wird, die ihre Affinität bzw. Avidität gegen Faktor VIII oder vWF auch unter stringenten Bedingungen beibehalten und der erhaltene Faktor VIII/vWF-Komplex auch unter diesen Bedingungen stabil bleibt.

Beispielsweise werden Antikörper ausgewählt, die das Antigen ohne Beeinträchtigung der Bindungseigenschaften bis zu 1 M NaSCN oder 0,5 M Guanidinhydrochlorid, oder sogar bis zu 100 % Sättigung Ammoniumsulfat binden. Jeweils 50% des Antigens werden noch bis zu 1,5 M NaSCN bzw. 0,75 M Guanidinhydrochlorid, 80 % Ethylenglykol, oder 0,75 M Harnstoff gebunden. Die für die Immunaffinitätschromatographie verwendeten Antikörper sind bevorzugterweise starke Antikörer, welche in einem Bindungstest an das immobilisierte Antigen auch aus verdünnten Lösungen von maximal 30 ng/ml, vorzugsweise maximal 15 ng/ml, binden, entsprechend einem Antikörper/Antigenverhältnis von 1:5 bis 1:20.

Aufgrund der hervorragenden Eigenschaften des erfindungsgemäß erhaltenen Komplexes in bezug auf seine Homogenität, insbesondere hinsichtlich der Faktor VIII-Bindungseigenschaften des enthaltenen vWF, ist er besonders geeignete zur Herstellung von pharmazeutischen Präparationen zur Verabreichung von Faktor VIII und/oder vWF. Es zeigte sich, daß dieser Komplex in einer gegenüber Plasma um mindestens 5000-fach, vorzugsweise 7100- bis 21 400-fach, angereicherten Konzentration gewonnen werden kann. Eine besondere Ausführungsform der vorliegenden Erfindung ist daher die Herstellung einer stabilen pharmazeutischen Präparation, enthaltend den hochgereinigten Komplex in einer gegenüber Plasma mindestens 5000-fach, vorzugsweise 7100- bis 21 400-fach, angereicherten Konzentration, wobei der Komplex eine hohe Komplex-Bindungsstärke aufweist und im wesentlichen frei von nicht-komplexiertem vWF bzw. Faktor VIII:C ist. Dadurch wird gewährleistet, daß kein überschüssiger vWF oder andere Proteine den Patienten belastet, bei Erhaltung der physiologischen Aktivität des Faktor VIII/vWF-Komplexes. Das erfindungsgemäß erhaltene Präparat zeichnet sich also durch seine hervorragende physiologische Akzeptanz aus.

Die Freiheit von nicht-komplexiertem vWF und/oder Faktor VIII:C bedeutet, daß weniger als 5 %, vorzugsweise weniger als 1 %, an freiem vWF bzw. Faktor VIII:C, bezogen auf den Gesamtproteingehalt, in der pharmazeutischen Präparation gefunden werden kann. Besonders bevorzugt sind Präparationen, bei welchen kein nicht-komplexierter vWF:Ag bzw. Faktor VIII:C nachgewiesen werden kann.

Der Nachweis von nicht-komplexiertem vWF bzw. Faktor VIII erfolgt durch Rechromatographie des erfindungsgemäß erhaltenen Komplexes am Immunaffinitätsmaterial, wobei dieses Material mit einer bestimmten Menge an freiem vWF bzw. Faktor VIII beladen wird, der Komplex erneut adsorbiert und in zuvor beschriebener Weise eluiert wird. Das nach Rechromatographie erhaltene Material enthält das gleiche Verhältnis Faktor VIII:C zur vWF:Ag wie im Ausgangsmaterial und zeigt daher keine relativen Verluste. Ein weiterer Test zum Nachweis von nicht-komplexgebundenem Faktor VIII und vWF ist die Bindung des Komplexes an immobilisierte Faktor VIII:C-Antikörper oder vWF-Antikörper, wobei nach der Adsorption kein wesentlichen Anteil, d.h. weniger als 5 %, an ungebundenem vWF bzw. Faktor VIII:C im Überstand nachgewiesen werden kann.

Die erfindungsgemäß erhaltene pharmazeutische Präparation zeichnet sich durch eine hohe Zuverlässigkeit bezüglich ihres Wirkungsspektrums aus, wobei das Risiko des Abbaus bzw. der Aktivierung des Faktor VIII durch den geringen Anteil an freiem Faktor VIII:C bzw. leicht-dissoziierbarem Faktor VIII:C erheblich gegenüber bekannten Faktor VIII:C/vWF-Komplexpräparationen reduziert ist.

Es versteht sich von selbst, daß die pharmazeutische Präparation, enthaltend den erfindungsgemäß erhaltenen Komplex, geeignete pharmazeutische Wirk-, Puffer-, Hilfs- oder Zusatzstoffe aufweisen kann, welche für Faktor VIII/vWF-Präparate verwendet werden. Aufgrund der ausgezeichneten Stabilität des Faktor VIII/vWF-Komplexes kann dieser aber auch ohne weitere Verwendung üblicher Stabilisatoren, wie Albumin, Zucker, insbesondere Trehalose oder Saccharose, zu einem stabilen pharmazeutischen Präparat verarbeitet werden.

Das erfindungsgemäß erhaltene pharmazeutische Präparat ist auch in Lösung bei neutralem pH ausreichend stabil, daß es als Flüssigpräparat bzw. flüssig-tiefgefrorenes Präparat zur Verfügung gestellt werden kann. Nach Rekonstitution eines entsprechenden lyophilisierten Präparates kann ebenfalls eine etwa gleichbleibende Zusammensetzung des Komplexes gezeigt werden.

Die zu verabreichende Dosis bzw. Konzentrationen des Komplexes im Präparat kann ebenfalls leicht vom Fachmann aufgrund der im Stand der Technik bekannten Verabreichungsregime für Faktor VIII/vWF-Präparate bestimmt werden.

Der Faktor VIII und/oder vWF kann im erfindungsgemäß erhaltenen Präparat als natives Protein, oder dessen Derivat, beispielsweise als durch Deletion, Substitution oder Insertion mutiertes Protein oder als chemisches Derivat bzw. als Fragment enthalten sein, sofeme die hohe Bindungsaffinität des Faktor VIII zu vWF im Komplex erhalten bleibt.

Gegenstand der vorliegenden Erfindung ist ein Verfahren zur immunaffinitätschromatographischen Reinigung eines Ausgangsmaterials, enthaltend Faktor VIII:C und vWF, welches durch die folgenden Schritte gekennzeichnet ist:
- Kontaktieren des Ausgangsmaterials mit einem Immunaffmitätschromatographie-Material,
- Eluieren von Faktor VIII/vWF-Komplex mit einem Elutionspuffer und
- weitere Elution von nicht-komplexiertem Faktor VIII:C bzw. vWF mit einem weiteren Elutionspuffer, welcher eine gegenüber dem vorigen Elutionspuffer erhöhte Konzentration bzw. Leitfähigkeit aufweist.

Durch die gezielte fraktionierte Eluierung der nicht-komplexierten Faktoren vom Komplex ist es erstmals gelungen, eine in Bezug auf eine bestimmte Bindungsstärke einheitliche von Willebrand-Faktor-Präparation bzw. einen Faktor VIII/vWF-Komplex zu erhalten.

Bevorzugterweise werden zur Immunaffinitätschromatographie Antikörper eingesetzt, die gegen den vWF gerichtet sind. Dadurch kann das Reinigungsverfahren, welches im wesentlichen auf die Fraktionierung unterschiedlicher vWF-Moleküle oder -Einheiten abzielt, noch mehr auf die unterschiedliche Natur des von Willebrand-Moleküls ausgerichtet werden.

Gemäß einer besonderen Ausführungsform des erfindungsgemäßen Verfahrens werden als Antikörper monoklonale Antikörper verwendet. Diese sind in bezug auf ihre Einheitlichkeit einem polyklonalen Antiserum vorzuziehen.

Ein bevorzugter Elutionspuffer für den erfindungsgemäß erhaltenen Komplex bei der Affinitätschromatographie ist ein Puffer enthaltend ein Thiocyanat und/oder ein Ammoniumsalz, vorzugsweise in einer Konzentration von nicht mehr als 2 M, am meisten bevorzugt im Bereich von 0,05 M bis 1,5 M. Dieser erste Elutionspuffer kann vorzugsweise Ethylenglykol, Glycerin oder ein Polyalkylenglykol enthalten.

Nicht-komplexierter Faktor VIII:C bzw. vWF werden bevorzugterweise mit einem Puffer enthaltend ein chaotropes Agens, insbesondere Thiocyanat, und/oder Ammoniumsalz, und/oder Alkalisalze und/oder Ethylenglykol, Glycerin oder ein Polyalkylenglykol eluiert und ebenfalls als homogene Präparationen gewonnen. Der Puffer weist dabei eine gegenüber dem ersten Elutionspuffer erhöhte Konzentration bzw. Leitfähigkeit auf, insbesondere eine um mehr als 20 %, vorzugsweise mehr als 50 % bis 100 % höhere Konzentration bzw. Leitfähigkeit.

Mit dem erfindungsgemäßen Verfahren wird auch eine von Willebrand-Präparation erhalten, welche eine verringerte Bindungs- bzw. Komplexierungskapazität gegenüber Faktor VIII:C aufweist.

Der hochgereinigte Komplex, aber auch die nicht-komplexierten Faktor VIII:C- bzw. vWF-Präparationen können gegebenenfalls durch weitere chromatographische Schritte, bevorzugterweise Ionenaustauscherchromatographie, Gelfiltration, hydrophobe Chromatographie, Affinitätschromatographie, insbesondere an immobilisiertem Heparin, oder Metallionenchelatchromatographie, weiter aufgereinigt und in bekannter und geeigneter Weise zu pharmazeutischen aber auch diagnostischen Präparaten aufgearbeitet werden.

Für die Herstellung von pharmazeutischen Präparaten ist es in der Regel notwendig, eine Behandlung zur Inaktivierung oder Abreicherung von Viren, vorzugsweise eine Hitzebehandlung und/oder eine physikalische bzw. chemische Behandlung, vorzusehen. Geeignete Behandlungsverfahren sind in der EP-0 159 311, der EP-0 519 901 sowie der WO 94/13329 beschrieben. Da der Komplex eine hohe Stabilität aufweist, wird diese Virusinaktivierungsbehandlung vorzugsweise am hochgereinigten Komplex durchgeführt.

Gemäß einer bevorzugten Ausführungsform des erfindungsgemäßen Verfahrens wird das Ausgangsmaterial ausgesucht aus der Gruppe bestehend aus Plasma, einer Plasmafraktion wie beispielsweise Kryopräzipitat oder einem Alkoholpräzipitat, einem Zellkulturüberstand und einem pharmazeutischen Präparat.

Die Erfindung wird anhand der folgenden Beispiele, auf die sie jedoch nicht eingeschränkt sein soll, näher erläutert.

### Beispiel 1: (derzeit nach Ansicht der Anmelderin der beste Weg zur Ausführung der beanspruchten Erfindung)

500 g Kryopräzipitat werden in Lösepuffer 1 + 4 bei 30°C gelöst. Danach wird die Lösung über ein 0,45 µm-Filter ( z.B. Millipore Durapore) geklärt. Eine mit 0,5 l anti-vWF-Gel (Fa. IMMUNOTECH, Frankreich) gefüllte Säule wird mit ca. 10 Säulenvolumina (= SV) Äquipuffer vorbereitet und darauf wird die geklärte Kryopräzipitatlösung mit 0,5 cm/min aufgetragen. Störende Verunreinigungen werden danach mit 5 bis 10 SV Äquipuffer (ev. mit erhöhter NaCl-Konzentration >250 mM) bei einer Flußrate von 1 cm/min ausgewaschen. Mit Elutionspuffer 1 wird dann der Faktor VIII/vWF-Komplex in einem ungefähren Verhältnis 1 : 1 eluiert. Die Elution mit Elutionspuffer 2 liefert vWF, der fast vollständig frei von Faktor VIII ist. Der zweite Elutionsschritt dient gleichzeitig zur Reinigung der Säule, sodaß danach sofort wieder äquilibriert werden kann.

| | | |
|---|---|---|
| **Lösepuffer** | 7,5 mM Tris | |
| | 60 mM NaCl | |
| | 100 mM Lysin | |
| | 100 mM Na-Acetat | pH 6,8 |
| | 25 E/ml Heparin | |
| **Äquipuffer** | 10 mM Tris | |
| | 100 mM NaCl | |
| | 100 mM Lysin | |
| | 3,25 mM CaCl₂ | pH 6,8 |
| **Elutionspuffer 1** | 10 mM Tris | |
| | 100 mM Glycin | |
| | 250 mM NaCl | |
| | 300 mM AMS | |
| | 3 mM CaCl₂ | |
| | 40 % Ethylenglykol | pH 6,5 |
| **Elutionspuffer 2** | 10 mM Tris | |
| | 100 mM Glycin | |
| | 1,25 M NaCl | |
| | 1,25 M NaSCN | |
| | 3 mM CaCl₂ | |
| | 40 % Ethylenglykol | pH 7,0 |

| **Ergebnisse:** | | | | | | | | |
|---|---|---|---|---|---|---|---|---|
| Probe | E/ml F VIII:C | %Ausb F VIII:C | E/mg F VIII:C | E/ml vWF | %Ausb vWF | E/mg vWF | F VIII:C zu vWF | Kollag.B. bez. vWF |
| Ausg. | 11,0 | 100,0% | 0,4 | 16,3 | 100,0% | 0,6 | 1:1,48 | 0,7 |
| F VIII/vWF | 12,7 | 50,9% | 133,7 | 14,7 | 39,8% | | 1:1,16 | 0,3 |
| vWF | 0,2 | 0,5% | | 23,4 | 37,2% | 236,4 | | 0,7 |

### Beispiel 2:

1 l fresh frozen Plasma wird bei T >25°C aufgetaut und danach 1 + 2 mit Äquipuffer verdünnt. Die Lösung wird durch ein 0,45 µm-Filter geklärt. Eine mit 50 ml anti-vWF-Gel (Fa. IMMUNOTECH) gefüllte Säule wird mit ca. 5 SV Äquipuffer vorbereitet und darauf die geklärte Plasmalösung mit einem Fluß von 1 cm/min aufgetragen. Die unerwünschten Verunreinigungen werden mit 10 bis 20 SV Äquipuffer + 250 mM NaCl ausgewaschen. Die erste Elution liefert Faktor VIII/vWF-Komplex; die zweite Elution vWF frei von Faktor VIII-Aktivität. Danach kann die Säule sofort wieder äquilibriert werden.

| | | |
|---|---|---|
| **Äquipuffer** | 5 g/l Na-Citrat | |
| | 2 E/ml Heparin | pH 7,0 |
| **Elutionspuffer 1** | 5 g/l Na-Citrat | |
| | 30 g/l AMS | |
| 20 g/l NaSCN | | |
| | 75 g/l Histidin | |
| | 5 g/l NaCl | |
| | 0,4 g/l CaCl₂ | pH 7,0 |
| **Elutionspuffer 2** | 5 g/l Na-Citrat | |
| | 50 g/l AMS | |
| | 50 g/l NaSCN | |
| | 75 g/l Histidin | |
| | 25 g/l NaCl | |
| | 0,4 g/l CaCl₂ | pH 7,0 |

| **Ergebnisse:** | | | | | | | | |
|---|---|---|---|---|---|---|---|---|
| Probe | E/ml F VIII C | %Ausb F VIII : C | E/mg F VIII | E/ml vWF | %Ausb vWF | E/mg vWF | F VIII:C zu vWF | Kollag.B. bez. vWF |
| **Ausg** | 0,3 | 100,0% | 0,01 | 0,3 | 100,0% | 0,01 | 1:1 | 1,0 |
| **F VIII/vWF** | 2,4 | 33,3% | 42,86 | 1,8 | 24,3% | | 1 : 0,75 | 0,5 |
| **vWF** | 0,0 | 0,2% | | 4,5 | 76,0% | 112,50 | | 0,8 |

## Patentansprüche

1. Verfahren zur immunaffinitätschromatographischen Reinigung eines Ausgangsmaterials, enthaltend Faktor VIII: C und von Willebrand-Faktor (vWF), **gekennzeiehnet durch** die folgenden Schritte:
- Kontaktieren des Ausgangsmaterials mit einem Immunaffinitätschromatographle-Material,
- Eluleren von Faktor VIII/vWF-Komplex mit einem Elutionspuffer und
- weitere Elution von nicht-komplexiertem Faktor VIII:C bzw. vWF mit einem weiteren Elutionspuffer, welcher eine gegenüber dem vorigen Elutionspuffer erhöhte Konzentration bzw. Leitfähigkeit aufweist.

2. Verfahren nach Anspruch 1, **dadurch gekennzeichnet, daß** zur Immunaffinitätschromatographie Antikörper eingesetzt werden, die gegen den vWF gerichtet sind.

3. Verfahren nach Anspruch 1 oder 2, **dadurch gekennzeichnet, daß** als Antikörper monoklonale Antikörper eingesetzt werden.

4. Verfahren nach einem der Ansprüche 1 bis 3, **dadurch gekennzeichnet, daß** die Antikörper mit einer hohen Affinität zu vWF eingesetzt werden, welche den vWF auch in einem Medium mit 1 M Thiocyanat binden können.

5. Verfahren nach einem der Ansprüche 1 bis 3, **dadurch gekennzeichnet, daß** der hochgereinigte Komplex mit einem Puffer, enthaltend ein Thiocyanat und/oder ein Ammoniumsalz, eluiert wird, vorzugsweise In einer Konzentration von höchstens 2 M, insbesondere im Bereich von 0,05 M - 1,5 M.

6. Verfahren nach einem der Ansprüche 1 bis 5, **dadurch gekennzeichnet, daß** der hochgereinigte Komplex mit einem Puffer, enthaltend Ethylenglykol, Glycerin oder ein Polyalkylenglykol, eluiert wird.

7. Verfahren nach einem der Ansprüche 1 bis 6, **dadurch gekennzeichnet, daß** nicht-komplexierter Faktor VII]:C bzw. vWF mit einem Puffer, enthaltend ein chaotropes Agens, insbesondere Thiocyanat und/oder Ammoniumsalz, und/oder Alkalisalze und/oder Glykole, wie Ethylenglykol, Glycerin oder ein Polyalkylenglykol, elulert und gewonnen wird, wobei der Puffer eine gegenüber dem Puffer aus Anspruch 5 oder 6 erhöhte Konzentration bzw. Leitfähigkeit aufweist.

8. Verfahren nach einem der Ansprüche 1 bis 7, **dadurch gekennzeichnet, daß** der hochgereinigte Komplex durch weitere chromatogrephische Schritte, vorzugsweise lonenaustauschchromatographle, Gelfiltration, hydrophobe Chromatographie, Affinitätschromatographie oder Metallionenchelatchromatographie, gereinigt wird.

9. Verfahren nach einem der Ansprüche 1 bis 8, **dadurch gekennzeichnet, daß** ein Behandlungschritt zur Inaktivierung bzw. Abreicherung von Viren, vorzugsweise einer Hitzebehandlung, vorgesehen wird, welcher vorzugsweise am hochgereinigten Komplex vorgenommen wird.

10. Verfahren nach einem der Ansprüche 1 bis 9, **dadurch gekennzeichnet, daß** als Ausgangsmaterial ein Mitglied aus der Gruppe, bestehend aus Plasma, einer Plasmafraktion, einem Zellkulturüberstand und einem pharmazeutischen Präparat, verwendet wird.

## Claims

1. A method for the immunoaffinity-chromatographic purification of a starting material comprising factor VIII:C and von Willebrand factor (vWF), **characterised by** the following steps:
- contacting the starting material with an immunoaffinity-chromatography material,
- eluting factor VIII/vWF-complex with an elution buffer, and
- further eluting non-complexed factor VIII:C or vWF, respectively, with a further elution buffer which, compared to the previous elution buffer, has a higher concentration and/or conductivity, respectively.

2. A method according to claim 1, **characterised in that** antibodies directed against vWF are used for said immunoaffinity-chromatography.

3. A method according to claim 1 or 2, **characterised in that** monoclonal antibodies are used as said antibodies.

4. A method according to any one of claims 1 to 3, **characterised in that** antibodies with a high affinity to vWF are used which are capable of binding vWF also in a medium with 1 M thiocyanate.

5. A method according to any one of claims 1 to 3, **characterised in that** the highly purified complex is eluted with a buffer containing a thiocyanate and/or an ammonium salt, preferably at a concentration of 2 M at the most, in particular in a range of from 0.05 M to 1.5 M.

6. A method according to any one of claims 1 to 5, **characterised in that** the highly purified complex is eluted with a buffer containing ethylene glycol, glycerol or a polyalkylene glycol.

7. A method according to any one of claims 1 to 6, **characterised in that** non-complexed factor VIII:C and/or vWF, respectively, is eluted and recovered with a buffer containing a chaotropic agent, in particular thiocyanate and/or ammonium salt, and/or alkaline salts and/or glycols, such as ethylene glycol, glycerol or a polyalkylene glycol, said buffer having an increased concentration and/or conductivity, respectively, compared to said buffer of claim 5 or 6.

8. A method according to any one of claims 1 to 7, **characterised in that** the highly purified complex is purified by further chromatographic steps, preferably ion exchange chromatography, gel filtration, hydrophobic chromatography, affinity chromatography or metal ion chelate chromatography.

9. A method according to any one of claims 1 to 8, **characterised in that** a treatment step for inactivating or depleting, respectively, viruses, preferably a heat treatment, is provided which preferably is carried out on the highly purified complex.

10. A method according to any one of claims 1 to 9, **characterised in that** as said starting material, a member from the group consisting of plasma, a plasma fraction, a cell culture supernatant and a pharmaceutical preparation is used.

## Revendications

1. Procédé pour la purification par chromatographie par immunoaffinité d'une substance de départ, contenant le facteur VIII:C et le facteur von Willebrand (vWF), **caractérisé par** les étapes suivantes :
- Mise en contact de la substance de départ avec un matériau de chromatographie par immunoaffinité,
- Elution du complexe du facteur VIII/vWF avec un tampon d'élution, et
- Elution additionnelle des facteurs non complexés VIII:C ou vWF avec un autre tampon d'élution, qui présente une concentration ou un pouvoir conducteur plus élevé par rapport au tampon d'élution précédent.

2. Procédé selon la revendication 1, **caractérisé en ce que** l'on utilise pour la chromatographie d'immunoaffinité, des anticorps qui sont dirigés contre le vWF.

3. Procédé selon la revendication 1 ou 2, **caractérisé en ce que** l'on utilise comme anticorps, des anticorps monoclonaux.

4. Procédé selon l'une quelconque des revendications 1 à 3, **caractérisé en ce que** les anticorps sont utilisés avec une affinité élevée pour le vWF, ceux-ci pouvant se lier au vWF également dans un milieu comprenant 1 M de thiocyanate.

5. Procédé selon l'une quelconque des revendications 1 à 3, **caractérisé en ce que** le complexe hautement purifié est élué avec un tampon contenant un thiocyanate et/ou un sel d'ammonium, de préférence en une concentration d'au plus 2 M, en particulier dans un domaine compris entre 0,05 M-1,5 M.

6. Procédé selon l'une quelconque des revendications 1 à 5, **caractérisé en ce que** le complexe hautement purifié est élué avec un tampon contenant de l'éthylène glycol, de la glycérine ou un polyalkylène glycol.

7. Procédé selon l'une quelconque des revendications 1 à 6, **caractérisé en ce que** le facteur VIII:C ou vWF non complexé est élué et obtenu avec un tampon contenant un agent chaotrope, en particulier un thiocyanate et/ou un sel d'ammonium et/ou des sels alcalins et/ou des glycols tels que l'éthylène glycol, la glycérine ou un polyalkylène glycol, le tampon présentant une concentration ou une conductivité accrues par rapport au tampon de la revendication 5 ou 6.

8. Procédé selon l'une quelconque des revendications 1 à 7, **caractérisé en ce que** le complexe hautement purifié est purifié par d'autres étapes chromatographiques, de préférence la chromatographie par échanges d'ions, la filtration sur gel, la chromatographie hydrophobe, la chromatographie d'affinités ou la chromatographie à ions métalliques.

9. Procédé selon l'une quelconque des revendications 1 à 8, **caractérisé en ce qu'**une étape de traitement est prévue pour l'inactivation ou l'affaiblissement de virus, de préférence un traitement thermique qui est entrepris de préférence sur le complexe hautement purifié.

10. Procédé selon l'une quelconque des revendications 1 à 9, **caractérisé en ce qu'**un membre du groupe constitué par le plasma, une fraction plasmatique, un résidu de culture cellulaire et une préparation pharmaceutiques est utilisé comme matériau de départ.
